# EUROPÄISCHE PATENTSCHRIFT

(11) **EP 0 984 745 B1**
(45) Veröffentlichungstag und Bekanntmachung des Hinweises auf die Patenterteilung: **01.10.2003**
(21) Anmeldenummer: 98934768.7
(22) Anmeldetag: 22.05.1998
(51) Int. Cl.: A61F 2/28, A61F 2/30, A61L 27/00

(54) **KNOCHEN- UND KNORPEL-ERSATZSTRUKTUREN**
REPLACEMENT STRUCTURES FOR BONES AND CARTILAGE
STRUCTURES POUR LE REMPLACEMENT DE L'OS OU DU CARTILAGE

(30) Priorität: 23.05.1997 DE 19721661
(43) Veröffentlichungstag der Anmeldung: 15.03.2000
(73) Patentinhaber: Jansson, V. Dipl.-Ing. Dr. Med ., D-82205 Gilching (DE); Zimmer, Markus, Dr., 83666 Waakirchen (DE)
(72) Erfinder: Jansson, V. Dipl.-Ing. Dr. Med ., D-82205 Gilching (DE); Zimmer, Markus, Dr., 83666 Waakirchen (DE)
(74) Vertreter: Czybulka, Uwe, Dipl.-Phys.
(86) Internationale Anmeldenummer: DE9801402
(87) Internationale Veröffentlichungsnummer: WO98052498

(56) Entgegenhaltungen:
- EP-A- 0 543 765
- EP-A- 0 560 279
- EP-A- 0 677 297
- WO-A-87/07495
- WO-A-88/03417
- WO-A-88/05312
- WO-A-94/09722
- WO-A-96/36562
- DE-A- 3 042 003
- US-A- 4 089 071
- US-A- 4 636 219
- US-A- 5 609 638

## Beschreibung

Die Erfindung betrifft ein Knochen-Knorpel-Implantat gemäß dem Oberbegriff des Patentanspruchs 1.

Es ist vor allem die Struktur derartiger Knochen-Knorpel-Implantate, welche die mechanischen und elastischen Eigenschaften der Werkstoffe bestimmt. Da diese Strukturen durch Auswahl der geeigneten physikalischen und geometrischen Parameter gezielt hergestellt werden können, ist es möglich, Implantate für den Ersatz von Knochen-, Knorpeln oder Knochen-Knorpel-Verbundimplantaten herzustellen, die den betreffenden natürlichen Knochen oder Knorpeln in ihren Elastizitäts-, Porösizitäts- und Festigkeitseigenschaften und damit auch dem biologischen Verhalten ihrer unmittelbaren Umgebung in vivo sehr nahe kommen.

In der Pathogenese der Arthrose aber auch bei posttraumatischen Zuständen und bei ausgelockerten Endoprothesen spielen Knochen- und Knorpeldefekte eine zentrale Rolle. Zur Deckung von Knochendefekten wird zum einen autologer oder homologer Knochen oder aber es werden Knochenersatzmaterialien verwendet. Während autologer Knochen nicht unbegrenzt zur Verfügung steht, müssen bei homologen Knochentransplantaten infektiologische Gesichtspunkte (z.B. HIV-Infektion) sowie logistische Probleme (Führung einer Knochenbank) bedacht werden. Auch ist die Verfügbarkeit homologen Knochens ebenfalls limitiert.

Aber auch Knochenersatzwerkstoffe sind nicht unproblematisch. Bedingt durch das Ausgangsmaterial (z. B. Korallen, Tierspongiosa) sind die gewonnenen Materialien sehr inhomogen und sehr klein. Materialien auf Hydroxylapatitbasis werden zudem praktisch nicht resorbiert und stellen auf Dauer einen Fremdkörper dar. Große Defekte lassen sich zudem nur durch Einlegen mehrerer Knochenersatzstücke decken, ein mechanisch belastbares Lager, z.B. zur Aufnahme einer Endoprothese, lässt sich so nur schwer erzielen. Zudem besteht bei den aus natürlichen Ausgangsmaterialien hergestellten Knochenersatzwerkstoffen ein weiteres grundsätzliches Problem: Der Knochen, der in die Poren des Ersatzmaterials einwächst, stellt die Negativstruktur des Ausgangsmaterials dar, wünschenswert wäre aber, wenn der neu gebildete Knochen die Architektur des Ersatzwerkstoffes annehmen könnte, die aber von der - nicht resorbierbaren - Ersatzstruktur belegt ist.

Aus einer Reihe von Untersuchungen ist außerdem bekannt, dass insbesondere die Porengröße für das Einwachsen des Knochens kritisch ist (Kühne J-H et al. 1994, Acta Orthop Scand. 65(3) :246-252). Ersatzmaterialien, bei denen die resultierende Porengröße eher das zufällige Resultat des Ausgangsproduktes oder des Verarbeitungsprozesses (z.B. Aufschäumen) ist, weisen daher oft .sehr schwankende Porengrößen auf. Auch das Verhältnis des Volumens der Ersatzstruktur zu dem freien Volumen, das dem Knochen zum Einwachsen zur Verfügung steht, ist bei diesen zufällig hergestellten Produkten ungünstig: Zu fordern ist, dass der Ersatzwerkstoff bei einer absolut offenporigen Grundstruktur mit minimalem Werkstoffvolumen nur Poren enthält, die bestimmte Größen nicht unter- bzw. überschreiten. Als minimale Porengröße dürften 150 µm, als maximale Größe 3 mm gelten. An den Stellen jedoch, an denen kein Knochenwachstum erfolgen soll (z.B. damit Blutgefäße in diese Struktur einsprossen können), muss in der Knochenersatzstruktur ein Kanalsystem mit entsprechend weitem Durchmesser angelegt werden, wobei hier als Maß 1 - 5 mm sinnvoll erscheinen. Außerdem ist zu fordern, dass die Knochenersatzstruktur gewissen mechanischen Anforderungen entspricht: Zum einen muss mit diesen Strukturen zwar direkt postoperativ eine mechanische Stabilität erreicht werden können (z.B. beim Einsatz dieser Strukturen beim Endoprothesenwechsel zur stabilen Verankerung eines erneut eingebrachten Implantates), zum anderen muss innerhalb der Struktur eine mechanische Belastung der einsprossenden Bindegewebszellen erfolgen, damit diese sich unter der Belastung zu tragfähigem Knochen differenzieren können. Der E-Modul dieser Strukturen sollte daher unter dem E-Modul des zu ersetzenden Knochen bleiben, diesen aber sinnvollerweise nicht wesentlich mehr als zu 60% unterschreiten. Da die Knochenstruktur jedoch sehr variabel ist und der E-Modul zwischen ca. 100 MPa (weicher spongiöser Knochen) und 20.000 MPa (harter kortikaler Knochen) schwankt, lassen sich diese Anforderungen mit den bisherigen Knochenersatzwerkstoffen nicht erfüllen.

Ein weiteres Problem stellen Knorpeldefekte dar. Dabei können Zerstörungen des Gelenkknorpels lokal begrenzt bleiben (z.B. Osteochondrosis dissecans) oder aber bei der manifesten Gelenkarthrose das gesamte Gelenk betreffen. In der Vergangenheit sind eine Reihe von Versuchen unternommen worden, Knorpeldefekte durch externe Züchtung von Knorpelzellen zu decken. Dabei werden patienteneigene Knorpelzellen entnommen, in Zellkultur vermehrt und anschließend in einer zweiten Operation in die Knorpeldefekte verbracht, indem sie dort mit geeigneten Bioklebern angeklebt oder aber unter einem über den Defekt vernähten Periostlappen verbracht werden. Außer dem Nachteil der zwei Operationen besteht bei dieser Methode das Problem, dass die Zellen am Ort der Gelenkschädigung verbleiben, dort angehen, wachsen und den Gelenkdefekt schließlich überbrücken müssen. Dieses ist oft nicht zu erreichen. Eine der Ursachen dürfte dabei sicher das zerstörte Knochenlager am Ort des Gelenkdefektes sein, an dem ja der ursprüngliche - und ursprünglich gesunde! - Knorpel ebenfalls nicht überlebt hat. Eine Übersicht über die zur Zeit diskutierten Verfahren zur Knorpelzelltransplantation findet sich in: Mesmer K, Gillquist J (1996) Cartilage Repair, a critical review. Acta Orthop Scand. 67(5) :523529.

Eigene Beobachtungen an explantierten gelockerten Knieendoprothesen zeigen jedoch ein hochinteressantes Phänomen: Kommt es bei Kniendoprothese nicht zu einer kompletten Auslockerung sondern zu einer Mikrolockerung des Implantates, bei der das Implantat noch in seiner Lage verbleibt, aber im Zehntelmillimeterbereich in seinem Auflager hin- und herrutscht, so kommt es bei den druckbelasteten Stellen zwischen Implantat und Knochen zu einer Umdifferenzierung des im Rahmen der Lockerung gebildeten Bindegewebes: Es bildet sich an diesen Stellen Knorpelgewebe, das von uns mit Hilfe immunhistochemischer Methoden teilweise als hyaliner Knorpel (entspricht Gelenkknorpel) gefunden wurde. Das ist insofern bemerkenswert, als nach bisheriger Überzeugung hyaliner Knorpel nicht neu entstehen kann, sondern bei reparativen Gelenkknorpelprozessen immer nur minderwertiger Faserknorpel entsteht. Diese Beobachtungen stehen aber durchaus im Gleichklang mit tierexperimentellen Untersuchungen: Wird in den Kondylus eines Kaninchenknies ein Loch in die Gelenkfläche gebohrt (sogen. "Leerlochversuch"), so bildet sich in diesem Loch aus dem einsprossenden Fasergewebe durchaus ein hyaliner Knorpel, der aber im Lauf weniger Wochen zu Faserknorpel degeneriert. Die Zusamenschau dieser Befunde mit den eigenen Beobachtungen lässt den Schluss zu, dass zur stabilen Ausdifferenzierung einer hyalinen Knorpelschicht eine bestimmte mechanische Belastung dieser Knorpelschicht erforderlich ist, die bei den Leerlochversuchen im Kaninchenkondylus nicht gegeben ist (fehlender mechanisch tragfähiger Unterbau, da der Knochen unterhalb der Knorpelschicht ebenfalls weggefräst wird), der aber bei den mikrogelockerten Prothesen jedoch gegeben war.

Diese Beobachtungen kann man in einem "Knorpelersatzmodell" umsetzen, indem man eine implantationsfähige Matrixstruktur erzeugt, die einerseits aus einer mechanisch belastbaren "Knochenersatzstruktur" sowie einer "Knorpelersatzstruktur" besteht. Es ist naheliegend zu vermuten, dass die mechanische Belastung der Zellen in diesen Schichten im physiologischen Bereich der Belastung des entsprechenden Gelenkes liegen sollte.

Eine prinzipielle Darstellung einer solchen "Gelenkersatzstruktur" findet sich auch schon in: Bittmann P, Müller W: Sulzer Technical Review, Info direct, Sulzer Innotec AG, Postfach 414, CH-8401 Winterthur, 1996. Die angegeben Strukturen sind jedoch unstrukturiert und für die externe Zellbeladung mit Knochen- und Knorpelzellen vorgesehen.

Zu fordern ist jedoch für den direkt implantierbaren "biologischen Gelenkersatz" eine Knochenersatzstruktur mit den oben angegebenen Forderungen mechanischer Simulation des Ersatzlagers mit den angesprochenen Problemen der Porengröße, Volumen der Ersatzstruktur sowie der dem Knochen angepassten mechanischen Steifigkeit, in Kombination mit einer "Knorpelersatzstruktur", die ganz bestimmte mechanische Eigenschaften erfüllen muss:
- definierter E-Modul unterhalb des E-Moduls hyaliner Gelenkschichten (ca. 34 MPa); vernünftigerweise sollte hier ein E-Modul (s.o.: Knochenersatzstruktur) von 0.2 - 1.0 des E-Moduls hyaliner Gelenkschichten angestrebt werden, damit die einsprossenden Stammzellen sich unter der mechanischen Belastung zu hyalinem Gelenkknorpel umdifferenzieren können.
- definierter mechanischer Unterbau der Knochenersatzstruktur, damit sofort nach Einsprossen der Stammzellen ein mechanisch wirksamer Druck auf die Zellen ausgeübt werden kann.
- eine möglichst hochgradig offenporige Struktur der Knorpelersatzstruktur, die insbesondere Raum zur Ausbildung der charakteristischen Säulenstruktur des hyalinen Gelenkknorpels mit der typischen bogenförmigen Faserstruktur lässt.

Vor allem aber ist eine höchstgradige "Struktureinheit" der Knorpelersatzstruktur mit exakt definierten mechanischen und geometrischen Eigenschaften wichtig. Zufällige geometrische Strukturen mit lokal zufällig wechselnden mechanischen Eigenschaften sind ungeeignet, da sich hyaliner Knorpel nur unter wohl definierten mechanischen Bedingungen innerhalb enger Grenzen bilden kann.

Da Knorpelzellen sich "in vivo" bilden können, ist der in Bittmann P, Müller W (1996) Sulzer (s.o.) beschriebene Weg des biologischen Gelenkersatzes mit externer Knorpelzüchtung und anschließender Beimpfung der Ersatzstrukturen mit diesen Zellen gar nicht erforderlich: Auch die in der Gelenkschicht angegebene homogene Füllung mit Agar (zum Anzüchten der Knorpelzellen in dieser Schicht) ist nicht erfprderlich, ja sogar schädlich, da sie (beim direkten biologischen Gelenkersatz) das Hineinlaufen des Knochenmarkblutes aus dem Knochenmark in diese Knorpelersatzstruktur und damit das Einsprossen der Stammzellen in diese Schicht nur verhindern würde.

Aus der EP-A1-0677297 ist ein Material für Implantate beschrieben, das eine gewebte oder gestrickte Grundstruktur aus organischen Fasern aufweist, wobei die Grundstruktur im wesentlichen durch relativ dicke Fasern gebildet ist, die durch entsprechend dünne Fasern umschlungen werden. Die Fasern sind hierbei aus biodegradierbarem Material und können zusätzlich zur besseren Verträglichkeit aus bioinertem Material bzw. mit einem solchen Material beschichtet sein. Zusätzlich kann die Oberfläche der organischen Fasern biologisch aktiviert werden, z. B. durch Beschichtung mit einem degradierbaren und absorbierbaren Polymer, das eine bioaktive Substanz enthält. Das Implantat kann in mehreren Achsrichtungen gewoben werden, um die mechanische Festigkeit an die Eigenschaften des zu ersetzenden biologischen Gewebes anzupassen.

Dieses bekannte Implantat aus einem Fasergewebe kann auch für den Ersatz von Knochengewebe verwendet werden.

Aus der US-A-4,089,071 ist ein Material für die Herstellung von Knochen-Endoprothesen bekannt, das aus einem Gewebenetz oder mehreren Gewebenetzen hergestellt ist, die z. B. aufgerollt werden können, wobei der dabei in den Netzen und zwischen den Netzen liegende Hohlraum durch einen Binder vollständig ausgefüllt ist. Die einzelnen Netze können hierbei in unterschiedlichen Richtungen ausgerichtet werden.

Auch bei einem derartigen Implantat sind die mechanischen Eigenschaften über die gesamte Struktur homogen; eine gewisse Variation dieser mechanischen Eigenschaften kann durch Kombination mehrerer Teilimplantate erreicht werden, die von einem weiteren Netz oder weiteren Netzen umwickelt werden.

Aus der DE-A1-3042003 ist ein Implantat bekannt, das insbesondere als Bewehrungsteil für zementöse Knochenfüllungen oder Knochenansätze mit zugeordneter Knochenzementmasse verwendet wird. Das Bewehrungsteil besteht aus einem weichelastischen, zurechtschneidbaren Schwamm aus köperverträglichem Material mit Kanaldurchmessern bis zu. einigen Millimetern, wobei diese Kanäle durch eine ausreichend dünnflüssige Knochenzement- oder sonstige schnellhärtende Kunststoffmasse völlig ausgefüllt werden. Der Schwamm besteht hierbei aus körperverträglichem Fadenmaterial.

Der Erfindung liegt die Aufgabe zugrunde, ein Knochen-Knorpel-Implantat anzugeben, dessen mechanische und strukturelle Eigenschaften optimal an das zu ersetzende Knochen- und Knorpelgewebe angepasst werden können.

Diese Aufgabe ist gemäß der Erfindung durch die Merkmale des Patentanspruchs 1 gelöst.

Die oben geschilderten Probleme des Knochen- und Knorpelersatzes sowie des biologischen Gelenkersatzes werden erfindungsgemäß gelöst, indem mit Hilfe eines geeigneten Herstellungsverfahrens geordnete Strukturen hergestellt werden, deren Eigenschaften bezüglich ihrer mechanischen Elastizität und Porosität definiert sind und den oben angegebenen Anforderungen entsprechen. Da es sich bei dieser vorgestellten Klasse von Ersatzwerkstoffen nicht mehr um "Werkstoffe" im klassischen Sinn handelt (der verwendete Werkstoff stellt nur eine der wesentlichen Eigenschaften, die geometrische Struktur die andere wesentliche Eigenschaft dar), wird im Folgenden nicht mehr von "Ersatzwerkstoffen" (wie bisher in der Literatur üblich) sondern von "Ersatzstrukturen" gesprochen, so wie dieses bei der Beschreibung der zu fordernden Strukturen bereits geschehen ist.

Gegenstand der Erfindung ist somit ein Knochen-Knorpelimplantat auf Basis eines dreidimensionalen Gitters bestehend im wesentlichen aus einer Vielzahl von regelmäßig angeordneten Stäben aus einem teilweise oder vollständig bioresorbierbaren Werkstoff, die eine geometrische dreidimensionale Struktur aus annähernd identischen Einheiten mit Zwischenräumen bilden, wobei besagte Struktur in Bezug auf Elastizität und Festigkeit auf das Gewebe abgestimmt ist, das es ersetzen soll.

Vorzugsweise ist Gegenstand der Erfindung ein Implantat nach Anspruch 1, welches dadurch gekennzeichnet ist, dass die Stäbe aus kugelförmigen oder zylindrischen Untereinheiten zusammengesetzt sind.

Vorzugsweise ist Gegenstand der Erfindung ein Implantat nach Anspruch 1, welches dadurch gekennzeichnet ist, dass die Stäbe aus einem der folgenden Werkstoffe bestehen: Poly-DLactide, Poly-L-Lactide, Poly-DL-Lactide, Hydroxylapatite, Calciumphosphate oder Mischungen, die im wesentlichen Calciumphosphate bzw. Hydroxylapatite enthalten, Collagen, Agar oder Gelatine.

Entsprechende Werkstoffe sind beispielsweise aus der WO 96/36562 oder der EP 0 543 765 bekannt. Auch entsprechende Werkstoffe aus resorbierbaren Polymermaterialien sind grundsätzlich geeignet

Vorzugsweise ist Gegenstand der Erfindung ein Implantat nach Anspruch 1, welches dadurch gekennzeichnet ist, dass die äußere Geometrie der Struktur des Implantats der Struktur des zu ersetzenden individuellen Knochen/Knorpel-Gewebes angepasst ist, so dass das Implantat aus Struktureinheiten mit regional unterschiedlichen geometrischen Parametern besteht. Die erforderlichen Daten können dabei aus der Struktur der Knochen bzw. Knorpel der einzelnen Patienten präoperativ mittels bekannter, ggf. computerunterstützter Methoden gewonnenen werden, z. B. mittels 3D-CTRekonstruktion.

Vorzugsweise ist Gegenstand der Erfindung ein Implantat nach Anspruch 1, welches dadurch gekennzeichnet ist, dass die Stäbe entsprechend der geforderten Festigkeit und Elastizität in den jeweiligen Bereichen des Implantats unterschiedliche Dicke besitzen und/oder dass die Abstände der Gitterstäbe in den jeweiligen Bereichen des Implantats unterschiedlich sind. Durch Auswahl der Dicke, des Werkstoffes und des Stababstandes können für einen bestimmten Bereich selektiv die notwendigen Eigenschaften erzeugt werden.

Vorzugsweise ist Gegenstand der Erfindung ein Implantat nach Anspruch 1, welches dadurch gekennzeichnet ist, dass das Elastizitätsmodul des Implantats mindestens 60 % des Elastizitätsmoduls des jeweiligen natürlichen Knochens und mindestens 20 % des jeweiligen natürlichen Knorpels ist. Wünschenswert sind jedoch Elastizitätsmodule, die nur um etwa 10 bis 30 % geringer sind als die des natürlichen Materials.

Das Knochen-Knorpelimplantat und insbesondere die Knochenersatzstruktur kann vorzugsweise durch ein dreidimensionales Gitter gebildet werden aus Einheiten von jeweils drei um etwa 90° zueinander versetzt angebrachten Stäben, wobei die so gebildete geometrische Struktur annähernd senkrecht zueinander stehende Ebenen von Stabreihen mit Zwischenräumen aufweist, in die natürliches Knochengewebe einwachsen kann, wobei eine der drei Stabreihen-Ebenen annähernd parallel zu der mechanischen Hauptspannungsrichtung des zu ersetzenden Knochens ist. Erfindungsgemäß sind auch solche Strukturen mit eingeschlossen, in welchen auch andere als rechte Winkel auftreten, vorzugsweise aber Strukturen mit Winkeln zwischen 60 und 90° mit den entsprechenden komplementären Winkeln.

Besonders geeignet sind auch sogenannte Scherengitterstrukturen.

Das Knochen-Knorpelimplantat und insbesondere die Knochenersatzstruktur kann durch ein dreidimensionales Gitter gebildet werden aus Einheiten von jeweils drei um etwa 90° zueinander versetzt angebrachten Stäben, wobei die so gebildete geometrische Struktur annähernd senkrecht zueinander stehende Ebenen von Stabreihen mit Zwischenräumen aufweist, in die natürliches Knochengewebe einwachsen kann, wobei eine der drei Stabreihen-Ebenen um etwa 45° zu der mechanischen Hauptspannungsrichtung des zu ersetzenden Knochens versetzt ist, und somit ein dreidimensionales Scherengitter vorliegt.

Die Knochenersatzstruktur weist vorzugsweise ein Elastizitätsmodul in einem Bereich von 50 bis 20.000 N/mm2 (= MPa) auf. Generell sollten diese Werte zwischen 30 und etwa 35.000 N/mm2 liegen, wobei bei Ersatz von spongiösen Knochen ein Elastizitätsmodul zwischen 30 und 200, vorzugsweise 60 und 100 N/mm2 und bei Ersatz von kortikalen Knochen entsprechende Werte zwischen 4.000 und 35.000 N/mm, vorzugsweise zwischen 5.000 und 20.000 N/mm2 vorliegen sollten.

Die Abstände zwischen den einzelnen Stäben in den erfindungsgemäßen Strukturen bilden Zwischenräume oder Poren, deren Durchmesser bestimmte Werte nicht unter- noch überschreiten sollte. Bei Knochenimplantaten liegt dieser Porendurchmesser zwischen 50 µm und 5mm, vorzugsweise zwischen 150 µm und 1 mm, bzw. zwischen 150 µm und 3 mm. In den erfindungsgemäßen Knochenersatzstrukturen sollten auch Bereiche vorgesehen sein, in die Gefäßzellen, wie z. B. Blutgefäße, einwachsen können. Die Durchmesser der Zwischenräume sollten hier zwischen 500 µm bis 5 mm, vorzugsweise zwischen 1 mm und 5 mm liegen.

Bei den Knochenersatzstrukturen weisen die von den Stäben gebildeten Zwischenräume (Poren) vorzugsweise einen Durchmesser von 150 µm bis 3 mm auf für Bereiche, in denen natürliches Knochengewebe einwachsen soll, und 1 bis 5 mm für Bereiche, in denen Gefäßzellen einwachsen sollen. Die Durchmesser der Stäbe können ebenfalls unterschiedlich sein. Erfindungsgemäß kann die Stabdicke nicht nur zwischen den verschiedenen Implantaten, sondern auch innerhalb eines Implantats, aber auch innerhalb eines einzelnen Stabes variieren. Die Auswahl der Stabdicke steht mit den geforderten physikalischen Eigenschaften des Implantats im Zusammenhang. Auch die Art des zur Verwendung kommenden Werkstoffes kann die Änderung der Stabdicke notwendig machen. Die Stabdicke variiert zwischen 10 µm und 3 mm, vorzugsweise zwischen 50 µm und 2mm, besonders bevorzugt zwischen 100 µm und 1mm.

Für die Knochenersatzstruktur liegt der Durchmesser der Stäbe bevorzugt zwischen 50 µm und 2 mm.

Die Knorpelersatzstruktur kann durch dreidimensionales Gitter gebildet wenden. aus Ebenen von jeweils um etwa 90° zueinander versetzt angebrachten, annähernd parallel auf Abstand liegenden Stäben, wobei die dadurch entstehende geometrische Struktur Zwischenräume aufweist, in die das natürliche Knorpelgewebe einwachsen kann, wobei die Anordnung der Stabreihen-Ebenen annähernd senkrecht zu der mechanischen Hauptspannungsrichtung der zu erwartenden Belastung ist. Insbesondere ist hierbei eine Stabreihenebene auf Lücke gesetzt im Vergleich zu einer benachbarten gleichausgerichteten Stabreihenebene.

Für die Knorpelersatzstruktur kann die dreidimensionale Gitterstruktur aus aufeinanderliegenden Stab-Gitterflächen gebildet werden.

Bei der Knorpelersatzstruktur können die Stab-Gitterflächen zueinander versetzt sein.

Die Knorpelersatzstrukturen sollten ein Elastizitätsmodul zwischen 5 und 40 N/mm² besitzen, vorzugsweise zwischen 7 und 35 N/mm².

Bei der Knorpelersatzstruktur weisen vorzugsweise die von den Stäben gebildetem Zwischenräume (Poren) einen Durchmesser von 5 µm bis 3 mm auf.

Der Durchmesser der Stäbe bei den Knorpelersatzstrukturen kann ebenfalls innerhalb einer Struktur und innerhalb eines Stabes variieren. Dabei sind Werte zwischen 1 µm und 2 mm, vorzugsweise zwischen 5 µm und 1 mm, insbesondere aber zwischen 10 µm und 300 µm anzustreben. Vorzugsweise liegt der Durchmesser der Stäbe zwischen 10 µm und 1 mm.

Vorzugsweise können die beiden beschriebenen Strukturen, insbesondere die harten Knochenstrukturen von weichen, vliesähnlichen Strukturen aus weichen Materialien wie Gelatine oder Agar überzogen sein.

Ferner können die beiden erfindungsgemäßen Strukturtypen miteinander zu einem Gelenkersatz-Implantat kombiniert werden, welches aus einem weicheren Überbau, der Knorpelstruktur, und einem härteren Unterbau, der Knochenstruktur, besteht.

Gemäß einer Vorteilhaften Ausgestalhung der Erfindung kann eine Gelenkersatzstruktur aufgebaut werden, bestehend im wesentlichen aus einem Knochenimplantat als Knochenersatzstruktur, wie oben, unten und in den Ansprüchen beschrieben, und einem Knorpelimplantat als Gelenkschicht-Struktur, wie oben, unten und in den Ansprüchen beschrieben, die in funktioneller Einheit verbunden sind.

Die erfindungsgemäßen Ersatzstrukturen können nach an sich bekannten Methoden, zum Beispiel durch Ausbohren oder Ausfräsen von soliden Blöcken hergestellt werden, wobei hier auch das Elekrodenstrahlbohren angewandt werden kann. Auch im Spritzgussverfahren können einfache Strukturen dieser Art hergestellt werden. Dort, wo Hinterschneidungen ein direktes Ausbohren oder Gussverfahren unmöglich machen, können die Bauteile geteilt hergestellt und nachträglich miteinander verbunden werden. Dieses Verfahren kann z.B. beim Aufbringen der Knorpelersatzstruktur vorteilhaft sein, wenn z.B. eine Vliesstruktur auf die Knochenersatzstruktur aufgebracht werden soll. Dabei können abbaubare Klebstoffe wie Fibrinkleber oder Gelate verwendet werden oder'aber, die Strukturen werden über eine Hitzebehandlung (z.B. Reibschweißen, thermisches Sintern) miteinander verbunden, sofern es sich um thermoplastische Werkstoffe handelt. Auf diese Weise können sogar große Strukturen aus einzelnen Stäben oder Substrukturen aufgebaut werden.

Es ist vorteilhaft, wenn die Strukturen mit stereolithograpischen Verfahren hergestellt werden, da so auch Strukturen aus einem Stück aufgebaut werden können, deren Hinterschneidungen durch ein bohrendes Verfahren nicht realisiert werden können. Mehrere Verfahren können dabei angewendet werden, die im Folgenden als "STL-Verfahren" bezeichnet werden, und von denen einige aufgeführt werden.

Beim sogenannten "Selective Laser Sintering" werden thermoplastische Werkstoffe pulverförmig Schicht für Schicht mit Hilfe eines Lasers verschmolzen. Die Konstruktionsdaten stammen aus einem CAD-Programm. Nachteilig bei diesem Verfahren ist, dass die Spurbreite des Lasers sehr feine Strukturen nicht ermöglicht und dass das freie, nicht verschmolzene Pulver aus den Hohlräumen der Struktur entfernt werden muss.

Besser geeignet erscheint daher das "Jet Phase Solidification" Verfahren. Hier wird mit einer feinen Spritzdüse ähnlich dem Tintenstrahldruckerprinzip der Werkstoff Spur um Spur und Schicht für Schicht aufgetragen. Entsprechend der Düse können so sehr feine Strukturen (zur Zeit bis 20µm Durchmesser) realisiert werden.

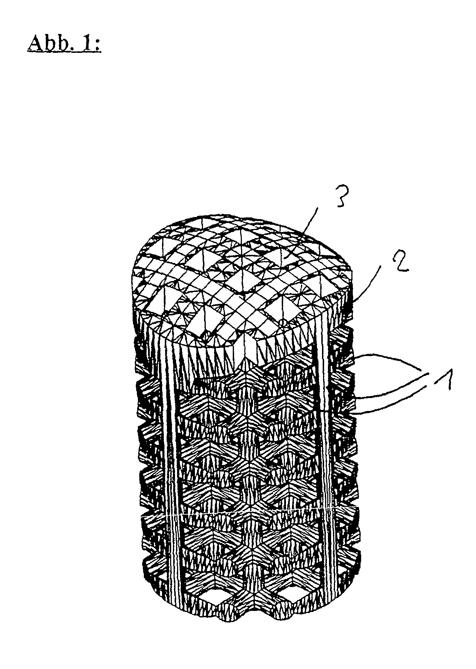

Als Knochenersatzstrukturen gut geeignet sind Strukturen aus Stabreihen (Fig.1) wobei zwei oder auch drei Stabreihen um jeweils 90° versetzt zueinander angebracht werden.
Entsprechend den mechanischen Anforderungen kann sich die Dicke der Stäbe sowie deren Ausrichtung innerhalb der Struktur auch ändern, so dass die trabekuläre Knochenstruktur des zu ersetzenden Knochens besser nachempfunden werden kann.

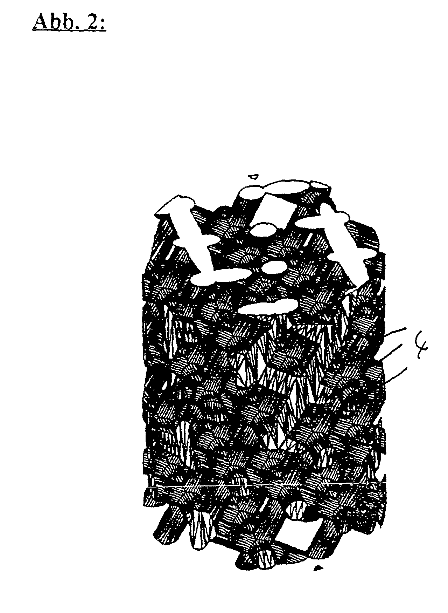

Günstig kann es auch sein, wenn die Gitterstruktur um 45° geneigt den mechanischen Hauptspannungstrajektorien im Sinne eines "Scherengitters" folgt, wie das in Fig.2 dargestellt ist: Im Vergleich mit den parallel zu den Hauptspannungstrajektorien angeordneten Stabstraktoren zeigen bei einer solchen versetzten Scherengitterstruktur größere Bereiche die gleiche Verformung unter einer äußeren mechanischen Belastung, da die senkrecht zu den Hauptspannungstrajektorien verlaufenden Stäbe kaum verformt werden. Eine möglichst gleiche Verformung der Knochenersatzstruktur unter Last ist jedoch zur gleichmäßigen Stimulation der anhaftenden Bindegewebszellen äußerst vorteilhaft.

Natürlich sind auch andere Grundmuster denkbar: So können z.B. aus aneinander haftenden (z.B. zusammengesinterten) Kügelchen Strukturen hergestellt werden, wobei durch Variation des Durchmessers der Kugeln oder deren Materialsteifigkeit die Struktursteifigkeit den örtlichen Gegebenheiten angepasst werden kann. Selbstverständlich sind auch alle anderen Grundmuster und deren Kombinationen denkbar, aus denen Knochen- und Knorpelersatzstrukturen mit definierten mechanischen und geometrischen Eigenschaften hergestellt werden können.

Besonders einfach und vorteilhaft werden solche Strukturen dann mit dem STL-Verfahren hergestellt. Dabei sind zur Simulation spongiösen Knochens E-Moduli der entstehenden Strukturen zwischen 100 und 5000 N/mm², zur Simulation kortikalen Knochens 5000 - 20.000 N/mm² anzustreben. Zum Erzielen eines hohen E-Moduls müssen den Matrixwerkstoffen ggf. Anteile an Zusatzstoffen wie Hydroxylapatit beigemengt werden, die - wie im Fall des Hydroxylapatits - auch noch osteoinduktive Eigenschaften besitzen.

Geeignete Matrixwerkstoffe sind insbesondere die Polyglycolide sowie Polylactide, insbesondere mit osteoinduktiven Beimischungen wie Hydroxylapatit, Tricalciumphosphat oder BMP ("bone morphogenic protein"). Ganz besonders gut für das Jet Phase Solidification Verfahren ist Poly-D-L-Lactid (50% D-Lactid, 50% L-Lactid), ein weitgehend amorphes Polylactid, das sich hervorragend spritzgießen (z.B. mit dem Jet Phase Solidification Verfahren) lässt und ein gutes biologisches Abbauverhalten zeigt, wobei die Abbauzeiten zwischen 3 - 12 Monaten liegen.

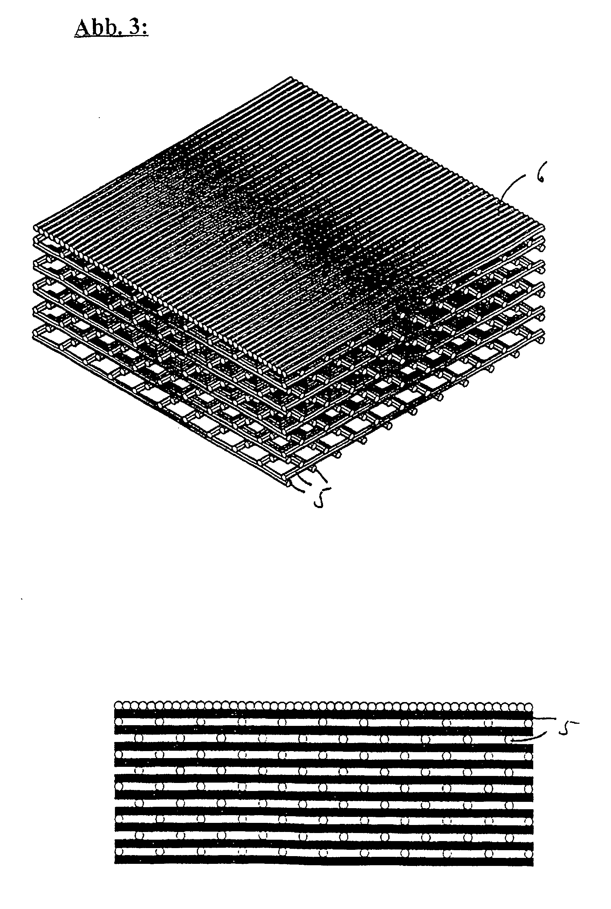

Als Knorpelersatzstruktur sind dagegen sehr viel weichere Schichten anzustreben. Sollen diese Strukturen ebenfalls aus Stäben hergestellt werden, so sind als Stabdicke je nach E-Modul des Werkstoffes und der verwendeten Stabkonstruktion Durchmesser von 5 µm bis 1 mm, die Abstände zwischen den Stäben Längen zwischen 5 µm - 3.0 mm anzustreben. Ein Beispiel einer solchen hochelastischen Schicht ist in Fig.3 angegeben: Hier sind zwei um 90° versetzte Stabreihen "auf Lücke" gesetzt worden, so dass eine extrem offenporige mechanisch weiche Matrix entsteht. Nimmt man als Beispiel reines Poly-L-D-Lactid mit einem E-Modul von ca. 5.000 MPa an, so lässt sich die Elastizität überschlägig einfach analytisch berechnen, wenn die Biegesteifigkeit der Stäbe, nicht jedoch die deren Quersteifigkeit sowie Zugsteifigkeit berücksichtigt wird. Danach lässt sich ein E-Modul von 34 MPa einer solchen Schicht bei einem vorgegebenen Stabdurchmesser von 30 µm und einem Abstand der Stäbe voneinander von ca. 0.15 mm erzielen. Berücksichtigt man die Tatsache, dass in einer solchen überschlägigen Rechnung die Struktur weicher gerechnet wird, als sie tatsächlich ist, und dass der E-Modul der Knorpelersatzstruktur ohnehin weicher als die physiologische Knorpelschicht sein sollte, so sollten die Abstände bei einem vorgegebenen Stabdurchmesser von 30 µm größer als 0.15 mm gewählt werden und ca. 0.2 - 0.6 mm betragen. Eine genauere Bestimmung des E-Moduls muss ohnehin für den jeweiligen Fall mit aufwendigeren Rechnungen (z.B. nach der Methode der finiten Elemente) oder experimentell an den fertigen Knorpelersatzstrukturen im Sinne einer Materialprüfung bestimmt werden.

Knorpelersatzstrukturen wie in dem genannten Beispiel (Fig.3) lassen sich besonders günstig im Jet Phase Solidification Verfahren herstellen, insbesondere, da eine solche Schicht direkt auf eine entsprechende Knochenersatzstruktur aufgebracht werden kann. Eine solche geordnet aufgebaute Struktur wie im genannten Beispiel aus 30 µm starken Stäben mit einem Versatz der Stäbe um 0.25 mm ergibt ein äußerst günstiges freies Verteilungsvolumen für die sich aufbauende Zellschicht von über 80%. Es kann sinnvoll sein, die dem Gelenk zugewandte Fläche der Knorpelersatzstruktur zu versiegeln. Dieses kann z.B. durch Auftragen einer dünnen Schicht aus Gelatine oder einem ähnlichen abbaubaren Werkstoff geschehen, oder aber es wird eine Folie z.B. aus Polyaminosäuren aufgeklebt oder aufgeschmolzen bzw. aufgesintert oder es werden eine oder mehrere Stabschichten dicht nebeneinander aufgebracht, wie in Fig.3 dargestellt ist. Diese Versiegelung hat zum einen den Sinn, das Abreiben der Stabstraktor zu verhindern, zum anderen hält es den Markblutkuchen in der Schicht und erhöht den auf diese Schicht wirkenden Druck.

Aus den oben beschriebenen Knochen- und Knorpelersatzstrukturen lassen sich nun Knochen- und Knorpeltransplantate sowie Strukturen für den "Direkten Biologischen Gelenkersatz" konstruieren.

### Knochenersatzstruktur als Knochentransplantat:

Dazu werden entweder kleinere Blöcke mit Kantenlängen zwischen 2mm und 20mm aus den oben beschriebenen Strukturen (z.B. entsprechend Fig. 1 und 2) im Sinne eines Granulats gefertigt, das in zu füllende Knochenhöhlen gelegt werden kann. Da im Allgemeinen spongiöse Defekte aufgefüllt werden müssen, bieten sich hierzu Struktursteifigkeiten an, die einem E-Modul des entstehenden Materials von 100 MPa bis 5.000 MPa entspricht. Sollen kortikale Defekte ausgefüllt werden, so sind ausnahmsweise auch Struktursteifigkeiten bis 20.000 Mpa erforderlich. Es kann sinnvoll sein, die äußere Form der Granulatkörnchen im Sinne sperriger Formen zu bauen, um eine Verzahnung der Granulatkörnchen untereinander zu erzielen, damit aus diesen Granulatkörnchen ein belastbares Knochenlager geformt werden kann, das sich z.B. zur Aufnahme eines Implantates bei einem Endoprothesenwechsel eignet.

Es ist aber auch denkbar, anstelle von Cranulatkörnern größere Defekte mit einem Stück auszufüllen, das z.B. intraoperativ aus einem großen Block herausgearbeitet wird. Besonders vorteilhaft ist es, wenn ein solches großes Stück präoperativ der Knochenhöhle angepasst wird, indem z.B. aus den bildgebenden Daten einer CT-Untersuchung der Knochenblock diesen Daten entsprechend individuell für den bestimmten Patienten angefertigt wird. Auf diese Weise ist es auch möglich, die Knochenersatzstruktur den lokalen Steifigkeiten des zu ersetzenden Knochenlagers I anzupassen, indem z. B. die Dicke der Stäbe (sofern eine Stabstraktor gewählt wird) sich ändert oder weitere Stäbe eingefügt werden.

### Knorpelersatzstruktur als Knorpeltransplantat:

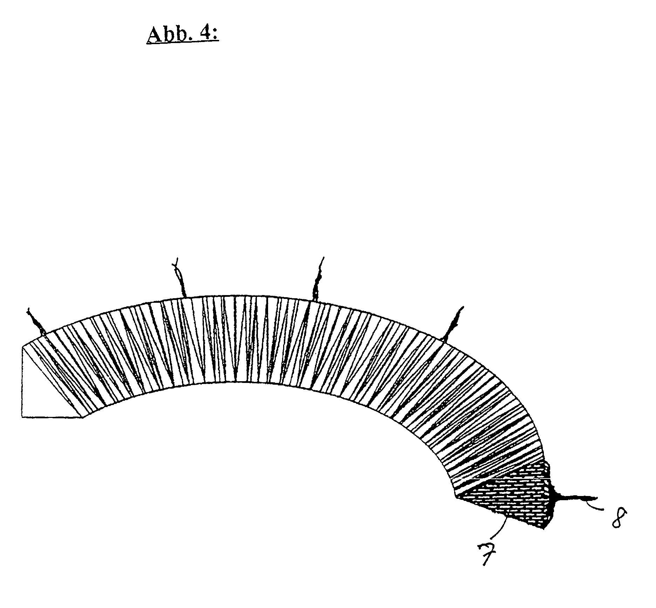

Eine Reihe von Anwendungen sind denkbar, bei denen aus einer wie oben beschriebenen Knorpelersatzstruktur ein Implantat gefertigt werden kann. So kann z.B. für einen verschlissenen Meniskus oder Diskus (z.B. discus triangularis im Handgelenk) eine Struktur gefertigt werden (Fig.4), die als Meniskustransplantat verwendet werden kann. Besonders vorteilhafterweise wird eine solche Struktur aus der oben erwähnten Stabstraktor entsprechend Fig.3 gefertigt, wobei die eine Stabreihe der halbmondförmigen Form des Meniskus folgen sollte, so dass die Form des Meniskus entsteht (Fig.4). Die Oberflächenversiegelung könnte hier, wie in Fig.3 dargestellt, durch eng aneinandergesetzte Stäbe einfach oder mehrschichtig erfolgen. Die Querstäbe könnten an der Meniskusbasis gebündelt und als Fäden ausgeleitet werden, an denen das Meniskustransplantat an der Gelenkkapsel verankert werden könnte.

Natürlich können durch Anpassung der Knorpelersatzstrukturen an die äußere Form auch andere Knorpelersatzimplantate gefertigt werden, z.B. für den schon oben erwähnten discus triangularis der Hand. Außer durch Bereitstellung verschiedener Standardgrößen kann natürlich auch individuell z.B. auf der Basis von CT-Daten eine individuelle Anpassung dieser Strukturen erfolgen.

### Biologischer Gelenkersatz:

Durch Kombination der Knochen- und der Knorpelersatzstruktur kann ein biologischer Gelenkersatz konstruiert werden. Dazu wird auf eine nicht zu weiche Knochenersatzstruktur (der E-Modul dieses "Unterbaus" sollte nur dicht unterhalb des E-Moduls des ursprünglichen Knochenlagers also, zwischen 45% -95% dieses E-Moduls liegen) eine Knorpelersatzstruktur im Bereich der Dicke der ursprünglichen Knorpeldicke aufgebracht. Dabei liegen diese Knorpeldicken im Bereich von 200 µm (z.B. Fingergelenk) bis ca. 4 mm (Hüftgelenk). Sinnvollerweise sollte die Knorpelersatzstruktur an der dem Gelenk zugewandten Fläche im Sinne der oben beschriebenen Verfahren gut versiegelt sein, schon allein deshalb, um ein Aufreiben der Neogelenkflächen zu verhindern, was insbesondere dann wichtig ist, wenn beide 15 Gelenkflächen ersetzt werden sollen.

Oft ist der Ersatz beider Gelenkfläche jedoch gar nicht erforderlich. So kommt es z.B. bei Ostechondrosis dissecans zu einer lokalisierten Knorpel/Knochenzerstörung nur der einen Gelenkseite. Der Durchmesser dieser Gelenkzerstörung ist begrenzt, so dass in diesen Fällen der Ersatz des Gelenkes mit Hilfe eines in der Größe und Gelenkkontur angepassten Zylinders erfolgen kann (Fig. 1, Fig. 2). Wie in Fig. 1 dargestellt, kann es sinnvoll sein, zwischen der Knochen- und Knorpelersatzstruktur eine "Supportschicht" (2) zu setzen, so dass die Knorpelersatzstruktur unter eine möglichst gleichmäßige Druckbelastung kommt. Auf die in Fig. 1 dargestellten Durchbrüche (3) kann dann verzichtet werden, wenn eine Zellbeladung der Knorpelstruktur vor dem Einsetzen in das Knochenlager erfolgt, z.B. durch introperative Impfung der Knorpelersatzstruktur mit Knochenmarkblut.

Sollen ganze Gelenke ersetzt werden, so ist außer der Form des Gelenkes natürlich die mechanische Struktursteifigkeit der Knochenersatzstruktur den mechanischen Gegebenheiten des zu ersetzenden Knochenlagers anzugleichen. Da die Knochensteifigkeiten physiologischerweise starken lokalen Schwankungen unterliegen, ist es günstig, wenn diese geometrischen Variationen mit einem geeigneten Herstellungsverfahren - wie z.B. mit dem geschilderten Jet Phase Solidification-Verfahren hergestellt werden.

Im Folgenden werden einige Strukturen an Hand von Abbildungen näher erläutert.

**Fig. 1:** Darstellung eines Zylinders als Knochenersatzstruktur. Die Knochenersatzstruktur ist aus um 90° zueinander versetzten Stäben dargestellt (1). Aufgesetzt wurde eine Supportstruktur (2), auf die eine (nicht dargestellte) Knorpelersatzstruktur aufgebracht werden kann. Damit Zellen aus dem Knochenmark in die Schicht der Knorpelersatzstruktur einwandern können, sind in der Supportstruktur Durchbrüche (3) vorgesehen. Mit einer derartigen Knorpelersatzstruktur wäre ein solcher Zylinder z.B. zum Ersatz eines lokalen einseitigen Knochen/Knorpeldefektes geeignet, wie er z.B. bei der osteochondrosis dissecans vorkommt.

**Fig. 2:** Darstellung einer Zylinders als Knochenersatzstruktur entsprechend Fig. 1. Die Stabreihen (4) sind in diesem Beispiel im Sinne eines "Scherengitters" angeordnet.

**Fig. 3:** Aufsicht und Seitenansicht einer Knorpelersatzstruktur aus feinen Stäben (5), die auf Lücke versetzt angeordnet wurden, um eine elastische Durchfederung der Schicht zu ermöglichen. Versiegelung der dem Gelenk zugewandten Oberfläche durch paralleles Aneinanderlegen der Stäbe (6).

**Fig. 4:** Darstellung der äußeren Form eines "Meniskustransplantates". Die Grundstruktur der Stabanordnung folgt dem in Fig.3 dargestellten Prinzip. Die Stabreihen (7) folgen der Halbmondform des Meniskustransplantates. Die dazu quer dazu verlaufenden Stäbe können an der Basis des Meniskustransplantates ausgeleitet und gebündelt werden, so dass das Meniskustransplantat mit diesen so entstehenden Fäden (8) im Gelenk fixiert werden kann.

## Patentansprüche

1. Knochen-Knorpelimplantat auf Basis eines dreidimensionalen Gitters bestehend aus einer Vielzahl von regelmäßig angeordneten Elementen aus einem teilweise oder vollständig bio-resorbierbaren Werkstoff, **dadurch gekennzeichnet, dass** das Implantat eine offenporige Knochenersatzstruktur und eine offenporige Knorpelersatzstruktur aus jeweils einem Gitter von Stäben (1; 4; 5, 6; 7) ist, **dass** die äußere Geometrie des Implantats der Form des zu ersetzenden Knochen- und Knorpel-Gewebes angepasst ist, und **dass** die Stäbe (1; 4; 5, 6; 7) entsprechend der geforderten Festigkeit und Elastizität in den jeweiligen Bereichen des Implantates unterschiedliche Dicke besitzen und/oder die Abstände der Stäbe (1; 4; 5, 6; 7) in den jeweiligen Bereichen des Implantats unterschiedlich sind, sodass das Implantat in Bezug auf Elastizität und Festigkeit auf das zu ersetzende Knochen- und Knorpelgewebe abgestimmt ist.

2. Implantat nach Anspruch 1, **dadurch gekennzeichnet, dass** das Implantat im Bereich der Knochenersatzstruktur einen Elastizitätsmodul von 50 bis 20.000 N/mm² und im Bereich der Knorpelersatzstruktur einen Elastizitätsmodul von 5 bis 40 N/mm² aufweist.

3. Implantat nach einem der vorhergehenden Ansprüche, **dadurch gekennzeichnet, dass** der Durchmesser der Stäbe (1; 4; 5, 6; 7) im Bereich der Knochenersatzstruktur des Implantates zwischen 50 Mikrometern und 2 Millimetern und im Bereich der Knorpelersatzstruktur zwischen 5 Mikrometern und 1 Millimeter liegt.

4. Implantat nach einem der vorhergehenden Ansprüche, **dadurch gekennzeichnet, dass** die durch die Zwischenräume zwischen den Stäben (1; 4; 5,6; 7) gebildeten offenen (3) Poren im Bereich der Knochenersatzstruktur, wo natürliches Knochengewebe einwachsen soll, einen Durchmesser von 150 Mikrometern bis 3 Millimeter und im Bereich der Knorpelersatzstruktur, wo Gefäßzellen einwachsen sollen, einen Durchmesser von 1 bis 5 Millimeter aufweisen.

5. Implantat nach einem der vorhergehenden Ansprüche, **dadurch gekennzeichnet, dass** auf die Knochenersatzstruktur und/oder die Knorpelersatzstruktur ein weiches Material, insbesondere eine vliesähnliche Struktur, Gelatine oder Agar, aufgebracht ist.

6. Implantat nach einem der vorhergehenden Ansprüche, **dadurch gekennzeichnet, dass** die Knorpelersatzstruktur zum Gelenk hin durch eine dünne Schicht versiegelt ist.

7. Implantat nach einem der vorhergehenden Ansprüche, **dadurch gekennzeichnet, dass** das dreidimensionale Gitter im Bereich der Knochenersatzstruktur und der Knorpelersatzstruktur ein orthogonales Gitter ist, in dem die Stäbe (1; 5; 7) jeweils um etwa 90° zueinander versetzt sind, wobei die Stäbe in einer der Stabreihenebenen der Knochenersatzstruktur so ausgerichtet sind, dass sie bei eingesetztem Implantat annähernd parallel zu der mechanischen Hauptspannungsrichtung des zu ersetzenden Knochens liegen.

8. Implantat nach Anspruch 6, **dadurch gekennzeichnet, dass** in einer der Stabreihenebenen der Knochenersatzstruktur die Stäbe (4) um etwa 45° zu der mechanischen Hauptspannungsrichtung versetzt sind, sodass ein dreidimensionales Scherengitter vorliegt.

9. Implantat nach einem der vorhergehenden Ansprüchen, **dadurch gekennzeichnet, dass** die Stäbe (1; 4; 5, 6;7) aus kugelförmigen oder zylindrischen Untereinheiten zusammengesetzt sind.

10. Implantat nach einem der vorhergehenden Ansprüche, **dadurch gekennzeichnet, dass** zwischen Knorpel- und Knochenersatzstruktur eine Supportstruktur (2) angeordnet ist, die Durchbrüche (3) aufweist, um eine Einwandern von Knochenmarkzellen durch die Supportstruktur in die Knorpelersatzstruktur zu erlauben.

11. Implantat nach einem der vorhergehenden Ansprüche, **dadurch gekennzeichnet, dass** die Stäbe (1; 4; 5, 6; 7) aus einem der folgenden Werkstoffe bestehen: Poly-D-Lactide, Poly-L-Lactide,Poly-DL-Lactide, Hydroxylapatide, Calciumphosphate oder Mischungen, die im wesentlichen Calciumphosphate bzw. Hydoxylapatite enthalten, Collagen, Agar oder Gelatine.

12. Verfahren zum Herstellen eines Implantates nach einem der vorhergehenden Ansprüche, **dadurch gekennzeichnet, dass** die zur Herstellung des Implantates erforderlichen Daten durch ein bildgebendes Verfahren bestimmt werden und die Knochen- und Knorpel-Ersatzstruktur entsprechend aufgebaut wird.

## Claims

1. Bone / cartilage implant on the basis of a three-dimensional grid, comprising numerous elements, in a regular arrangement, of a material that is partially or fully bio-absorbable,
**characterised in that**
the implant is an open-pore bone replacement structure and an open-pore cartilage replacement structure of, respectively, a grid of rods (1; 4; 5, 6; 7); that the outer geometry of the implant is adapted to the shape of the bone tissue and cartilage tissue that is to be replaced;
and that in accordance with the required strength and elasticity in the respective areas of the implant, the rods (1; 4; 5, 6; 7) are of varying thickness and/or the distances between the rods (1; 4; 5, 6; 7) vary in the respective areas of the implant, so that in respect of elasticity and strength, the implant is matched to the bone tissue and cartilage tissue that is to be replaced.

2. Implant in accordance with claim 1, **characterised in that** the implant has a modulus of elasticity of 50 to 20,000 N/mm² in the area of the bone replacement structure, and a modulus of elasticity of 5 to 40 N/mm² in the area of the cartilage replacement structure.

3. Implant in accordance with one of the preceding claims, **characterised in that** the diameter of the rods (1; 4; 5, 6; 7) lies between 50 micrometres and 2 millimetres in the area of the bone replacement structure of the implant, and between 5 micrometres and 1 millimetre in the area of the cartilage replacement structure.

4. Implant in accordance with one of the preceding claims, **characterised in that** in the area of the bone replacement structure, where natural bone tissue is to grow in, open (3) pores formed through the spaces between the rods (1; 4; 5, 6; 7) have a diameter of 150 micrometres to 3 millimetres, and a diameter of 1 to 5 millimetres in the area of the cartilage replacement structures, where vascular cells are to grow in.

5. Implant in accordance with one of the preceding claims, **characterised in that** a soft material, in particular a structure like a non-woven fabric, gelatine or agar, is applied to the bone replacement structure and/or the cartilage replacement structure.

6. Implant in accordance with one of the preceding claims, **characterised in that** relative to the joint, the cartilage replacement structure is sealed off by a thin layer.

7. Implant in accordance with one of the preceding claims, **characterised in that** in the area of the bone replacement structure and the cartilage replacement structure, the three-dimensional grid is an orthogonal grid, in which the rods (1; 5; 7) are respectively offset against one another by around 90°, wherein the rods in one of the rod row planes of the bone replacement structure are aligned in such a way that when the implant is deployed, they lie approximately parallel to the mechanical main direction of tension of the bone that is to be replaced.

8. Implant in accordance with claim 6, **characterised in that** in one of the rod row planes of the bone replacement structure, the rods (4) are offset by around 45° to the mechanical main direction of tension, so that a three-dimensional scissor grid is achieved.

9. Implant in accordance with one of the preceding claims, **characterised in that** the rods (1; 4; 5, 6; 7) are composed of spherical or cylindrical sub-units.

10. Implant in accordance with one of the preceding claims, **characterised in that** arranged between the replacement structure for bone and cartilage is a support structure (2) that has break-outs (3) to permit the immigration of marrow cells through the support structure into the cartilage replacement structure.

11. Implant in accordance with one of the preceding claims, **characterised in that** the rods (1; 4; 5, 6; 7) comprise one of the following materials: poly-D-lactide, poly-L-lactide, poly-DL-lactide, hydroxyl apatide, calcium phosphate or blends that essentially contain calcium phosphate or hydroxyl apatite, collagen, agar or gelatine.

12. Method for producing an implant in accordance with one of the preceding claims, **characterised in that** the data required for producing the implant are determined through an illustrative method, and the replacement structure for bone and cartilage is constructed accordingly.

## Revendications

1. Implant d'os ou de cartilage à base d'un treillis tridimensionnel formé de plusieurs éléments disposés de manière régulière d'un matériau partiellement ou totalement bio-résorbant, **caractérisé en ce que** l'implant présente une structure de remplacement d'os à pores ouverts et une structure de remplacement de cartilage à pores ouverts formées chacune d'un treillis de barres (1 ; 4 ; 5, 6 ; 7), **en ce que** la géométrie extérieure de l'implant est adaptée à la forme du tissu de l'os ou du cartilage à remplacer, et **en ce que** les barres (1 ; 4 ; 5, 6 ; 7) ont une épaisseur différente selon la solidité et l'élasticité requises dans les diverses zones de l'implant et/ou les espacements des barres (1 ; 4 ; 5, 6 ; 7) sont différents dans les diverses zones de l'implant, de sorte que l'implant est adapté à l'élasticité et à la solidité du tissu de l'os ou du cartilage à remplacer.

2. Implant selon la revendication 1, **caractérisé en ce que** l'implant présente un module d'élasticité de 50 à 20 000 N/mm² dans la zone de la structure de remplacement d'os et un module d'élasticité de 5 à 40 N/mm² dans la zone de la structure de remplacement de cartilage.

3. Implant selon l'une des revendications précédentes, **caractérisé en ce que** le diamètre des barres (1 ; 4 ; 5, 6 ; 7) est compris entre 50 micromètres et 2 millimètres dans la zone de la structure de remplacement d'os de l'implant et entre 5 micromètres et 1 millimètre dans la zone de la structure de remplacement de cartilage.

4. Implant selon l'une des revendications précédentes, **caractérisé en ce que** les pores ouverts (3) formés par les espaces entre les barres (1 ; 4 ; 5, 6 ; 7) présentent un diamètre de 150 micromètres à 3 millimètres dans la zone de la structure de remplacement d'os, où du tissu d'os naturel doit pouvoir s'insérer, et un diamètre de 1 à 5 millimètres dans la zone de la structure de remplacement de cartilage, où des cellules de vaisseaux doivent pouvoir s'insérer.

5. Implant selon l'une des revendications précédentes, **caractérisé en ce qu'**un matériau mou, en particulier une structure formant couche, gélatine ou agar, est disposé sur la structure de remplacement d'os et/ou la structure de remplacement de cartilage.

6. Implant selon l'une des revendications précédentes, **caractérisé en ce que** la structure de remplacement de cartilage est recouverte d'une couche mince au niveau de l'articulation.

7. Implant selon l'une des revendications précédentes, **caractérisé en ce que** le treillis tridimensionnel est un treillis orthogonal dans la zone de la structure de remplacement d'os et dans la zone de structure de remplacement de cartilage, dans lequel les barres (1 ; 5 ; 7) sont décalées chacune d'environ 90° par rapport à l'autre, les barres d'un plan de rangées de barres de la structure de remplacement d'os étant disposées de façon à être, lorsque l'implant est inséré, sensiblement parallèles à la direction de contrainte maximale de l'os à remplacer.

8. Implant selon la revendication 6, **caractérisé en ce que**, dans un plan de rangées de barres de la structure de remplacement d'os les barres (4) sont décalées d'environ 45° par rapport à la direction de contrainte maximale, de façon à produire un treillis à ciseaux tridimensionnel.

9. Implant selon l'une des revendications précédentes, **caractérisé en ce que** les barres (1 ; 4 ; 5, 6 ; 7) sont rassemblées en sous-unités coniques ou cylindriques.

10. Implant selon l'une des revendications précédentes, **caractérisé en ce qu'**une structure support (2) est agencée entre la structure de remplacement de cartilage et la structure de remplacement d'os, la structure support présentant des ouvertures (3) pour permettre une intrusion de cellules de moelle osseuse dans la structure de remplacement de cartilage à travers la structure support.

11. Implant selon l'une des revendications précédentes, **caractérisé en ce que** les barres (1 ; 4 ; 5, 6 ; 7) sont formées d'un des matériaux suivants : poly-D-lactide, poly-L-lactide, poly-DL-lactide, hydroxylapatite ("Hydroxylapatide"), phosphate de calcium ou de mélanges comprenant pour l'essentiel du phosphate de calcium ou de l'hydoxylapatite, collagène, agar ou gélatine.

12. Procédé pour fabriquer un implant selon l'une des revendications précédentes, **caractérisé en ce que** les données nécessaires à la fabrication de l'implant sont déterminées par un procédé graphique et les structures de remplacement d'os et de cartilage sont réalisées de façon correspondante.
